# EUROPEAN PATENT APPLICATION

(11) **EP 0 652 011 A1**
(43) Date of publication of application: **10.05.1995**
(21) Application number: 93913569.5
(22) Date of filing: 23.06.1993
(51) Int. Cl.: A61K 31/685, A61K 37/02, A61K 37/22, A61K 35/42

(54) **PROPHYLACTIC AND REMEDY FOR VIRAL DISEASES IN RESPIRATORY TRACT**

(30) Priority: 24.06.1992 JP 165875/92
(71) Applicant: TOKYO TANABE COMPANY LIMITED, Chuo-ku Tokyo 103 (JP)
(72) Inventor: KIDO, Hiroshi, Tokushima 770 (JP); TASHIRO, Masato 4-101, Jichiidai-jutaku, Tochigi 323 (JP); SAKAI, Kentaro 232-1, Otsubo, Tokushima 770 (JP); SEKIDO, Shozaburo, Chiba 279 (JP)
(74) Representative: Skailes, Humphrey John
(86) International application number: JP9300851
(87) International publication number: WO9400131

(57) **Abstract**

A prophylactic and a remedy for viral diseases in the respiratory tract, each containing a lung surfactant as the active ingredient and being applicable to the prevention or treatment of diseases caused by a virus which contains an adventitial glycoprotein and which epidemically infects the respiratory tract and proliferates therein, such as influenza virus, parainfluenza virus, RS virus, measles virus or mumps virus.

## Description

### FIELD OF THE INVENTION

This invention relates to a prophylactic and therapeutic agent for viral diseases in a region of the respiratory tract which contains a pulmonary surfactant (hereinafter referred to as "PSF"). More particularly, it relates to an agent containing a PSF as active ingredient and useful for the prevention and treatment of viral diseases in a region of the respiratory tract where bronchial mucoepithelial secretory cells are interspersed.

### BACKGROUND ART

Bronchial mucoepithelial secretory cells are interspersed in a region of the respiratory tract, especially in a bronchial region. These cells have the function of secreting airway mucus and, moreover, is said to be partly involved in the production of an in vivo pulmonary surface-active substance. On the other hand, a PSF is a substance comprising, as a principal component, a phospholipid consisting essentially of a phosphatidyl-choline (hereinafter sometimes referred to as "choline phosphoglyceride"), and a great variety of PSFs have been reported as will be described later. According to those reports, PSFs generally have the effect of reducing the surface tension of the gas-liquid interface in pulmonary alveoli, maintaining the alveolar cavities physiologically, and thereby smoothing the respiratory function of the lungs. Accordingly, they are accepted as useful therapeutic agents for respiratory distress syndrome that is a highly fatal disease caused by a lack of the in vivo pulmonary surface-active substance. It has also be reported in International Publication WO91/17766 that PSFs have the effect of stabilizing the bronchi and suppressing allergic bronchoconstriction and, therefore, are useful as therapeutic agents for asthma.

However, little is known about the role of the bronchial mucoepithelial secretory cells in the living body, and no distinct relationship between the bronchial mucoepithelial secretory cells and viral diseases or between these cells and the in vivo pulmonary surface-active substance has been elucidated as yet. Accordingly, the effectiveness of PSFs in viral diseases has not been known.

The present inventors have carried on investigations on proteases and viruses for many years and have now found that (1) a trypsin-like protease (hereinafter referred to tentatively as "Tryptase Clara") is secreted from the bronchial mucoepithelial secretory cells, as discovered by the present inventors; (2) Tryptase Clara limitedly decomposes virus envelope glycoprotein precursors to mature type virus envelope glycoproteins having membrane fusion activity, thereby inducing fusion of the virus envelope and the cell membrane in a region of the respiratory tract and determining the entry of viruses into bronchial mucoepithelial cells; (3) the in vivo pulmonary surface-active substance suppresses the maturation of viruses induced by the decomposition of virus envelope glycoprotein precursors by Tryptase Clara, and thereby prevents the infection and multiplication of viruses in bronchial mucoepithelial cells; (4) as a result of the prevention of virus infection and multiplication, the prevention and treatment of viral diseases which may be accompanied by severe fever or inflammation or by superinfection with bacteria can be achieved; and (5) externally administered PSFs also have the same effects as the in vivo pulmonary surface-active substance. The present invention has been completed on the basis of these findings.

### DISCLOSURE OF THE INVENTION

The present invention provides a prophylactic and therapeutic agent for viral diseases which contains a PSF as active ingredient.

The PSF used in the prophylactic and therapeutic agent for viral diseases in accordance with the present invention may be a substance containing a phospholipid consisting essentially of a phosphatidylcholine, in a total amount of not less than 40% by weight. More specifically, the following well-known or novel PSFs are useful.
(a) A surface-active substance comprising phospholipid, neutral lipid, total cholesterol, carbohydrate and protein that are present in the lung tissue of a mammal, the weight percentages of these components based on the total weight of the dried final product being 75.0-95.5% for the phospholipid, 1.8-14.0% for the neutral lipid, not greater than 3.0% for the total cholesterol, 0.1-1.5% for the carbohydrate, and not greater than 5.0% for the protein (Japanese Patent Publication No. 9925/'86).
(b) A pulmonary surface-active pharmaceutical composition consisting essentially of dipalmitoyl-phosphatidylcholine and a fatty alcohol (Japanese Patent Laid-Open No. 99524/'82).
(c) A surface-active substance comprising phospholipid, neutral fat, total cholesterol, free fatty acids, carbohydrate and protein that are present in the lung tissue of a mammal, the weight percentages of these components based on the total dry weight of the substance being 68.6-90.7% for the phospholipid, 0.3-13.0% for the neutral fat, 0.0-8.0% for the total cholesterol, 1.0-27.7% for the free fatty acids, 0.1 - 2.0% for the carbohydrate, and 0.0-3.5% for the protein (hereinafter referred to as "PSF-1"; Japanese Patent Publication No. 9924/'86).
(d) A synthetic pulmonary surface-active substance consisting essentially of phosphatidylcholine that is a phospholipid, and an unsaturated fatty acid or an ester thereof, the phosphatidylcholine comprising 55-80% by weight of the whole substance (Japanese Patent Laid - Open No. 135813/'83).
(e) A pulmonary surface-active substance comprising not less than 80% by weight of phospholipid based on the total weight thereof and containing essentially no protein (Japanese Patent Laid-Open No. 164513/'83).
(f) A pulmonary surface-active substance comprising phospholipid, neutral lipid, total cholesterol and carbohydrate that are extracted from the lung of a mammal, and containing essentially no protein, the composition of the dried product consisting of 70-95% by weight of the phospholipid, 1-10% by weight of the neutral lipid, not greater than 3.0% by weight of the total cholesterol, and not greater than 0.3% by weight of the carbohydrate (Japanese Patent Laid-Open No. 183620/'83).
(g) A synthetic pulmonary surface-active substance consisting essentially of phosphatidylcholine and cardiolipin that are phospholipids, the phosphatidylcholine comprising 55-80% by weight of the whole substance (Japanese Patent Publication No. 29171/'89).
(h) A pulmonary surface-active agent comprising 40-45% by weight of dipalmitoylphosphatidylcholine, 5-10% by weight of dipalmitoylphosphatidylglycerol and 50% by weight of a sugar (Japanese Patent Publication No. 13690/'89).
(i) A synthetic pulmonary surface-active substance comprising, based on the total weight thereof, 80-95% by weight of phospholipid consisting of phosphatidylcholine and cardiolipin and/or phosphatidylglycerol, 5-20% by weight of neutral lipid, and 0-10% by weight of a fatty acid (Japanese Patent Laid-Open No. 95219/'84; Journal of the Japanese Society for Surface Medicine, Vol. 14, No. 1, p. 59, 1983).
(j) A surfactant consisting essentially of a choline phosphoglyceride, an acid phospholipid, a fatty acid or its analogue, and a lipoprotein derived from the lung of a mammal, the contents of these components based on the total weight of the surfactant being 50.6-85.0% by weight for the choline phosphoglyceride, 4.5-37.6% by weight for the acid phospholipid, 4.6-24.6% by weight for the fatty acid or its analogue, and 0.1-10.0% by weight for the lipoprotein (hereinafter referred to as "PSF-2"; Japanese Patent Publication No. 78371/'91).
(k) A synthetic pulmonary surface-active substance comprising, based on the total weight thereof, 55-80% by weight of a phosphatidylcholine having two saturated straight-chain fatty acid residues, 10-35% by weight of a phosphatidylglycerol having two saturated straight-chain fatty acid residues, and 5-20% by weight of neutral lipid (Japanese Patent Laid-Open No. 181216/'84).
(l) An agent mixture comprising 40-70% of phospholipid, less than 1.5% of protein, 10-40% of cholesterol, and 5-30% of neutral lipid (Japanese Patent Laid-Open No. 237023/'85).
(m) A surfactant consisting essentially of a choline phosphoglyceride, an acid phospholipid and a fatty acid or its analogue, the contents of these components based on the total weight of the surfactant being 53.9-87.8% by weight for the choline phosphoglyceride, 4.8-38.2% by weight for the acid phospholipid, and 7.0-26.2% by weight for the fatty acid or its analogue (hereinafter referred to as "PSF-3"; Japanese Patent Publication No. 8768/'90).
(n) A pulmonary surface-active substance comprising lipid extracted from the alveolar lavage fluid of pigs and calcium chloride added thereto (Journal of the Japanese Society for Surface Medicine, Vol. 12, No. 1, p. 1, 1981; ibid., vol. 14, No. 2, p. 12, 1983).
(o) A synthetic pulmonary surface-active substance comprising a three-component mixture of dipalmitoylphosphatidylcholine, distearoylphosphatidylcholine and soybean lecithin (Japanese Patent Publication No. 9292/'89).
(p) A pulmonary artery surface-active agent of animal origin comprising a polar lipid fraction and a protein fraction, the polar lipid fraction being present in an amount of at least 98.5% by weight and consisting essentially of at least 95% of a phospholipid mixture (Japanese Patent Laid-Open No. 63526/'89).
(q) Synthetic pulmonary surface-active substances comprising a phospholipid, and a pulmonary surface-active protein as described in National Publication No. 501122/'87, National Publication No. 501792/'87, National Publication No. 503222/'88, National Publication No. 501282/'89, Japanese Patent Laid-Open No. 424/'90, Japanese Patent Laid-Open No. 6405/'90, Japanese Patent Laid-Open No. 53798/'90, Japanese Patent Laid-Open No. 279628/'90, National Publication No. 502917/'90, Japanese Patent Laid-Open No. 44332/'91 or Japanese Patent Laid-Open No. 90033/'91, or other pulmonary surface-active protein prepared by gene recombination.
(r) Natural pulmonary surface-active substances such as Alveofact (trade name; hereinafter referred to as "PSF-4") obtained from cattle lung and comprising phospholipid, cholesterol, hydrophobic surface-active protein, free fatty acids and triglycerides, Infasurf (trade name), Curosurf (trade name) and Humansurf (trade name) obtained from human amnionic fluid, and preparations thereof.
(s) Synthetic pulmonary surface-active substances such as Exosurf (trade name; hereinafter referred to as "PSF-5") and Alec (trade name) comprising 7 parts of dipalmitoyl-phosphatidylcholine and 3 parts of phosphatidylglycerol.
(t) A pulmonary surface-active substance consisting essentially of a choline phosphoglyceride, an acid phospholipid, a fatty acid or its analogue, and a peptide represented by the following specific sequence, the contents of these components based on the total dry weight of the substance being 50.6-85.0% by weight for the choline phosphoglyceride, 4.5-37.6% by weight for the acid phospholipid, 4.6-24.6% by weight for the fatty acid or its analogue, and 0.1-5.0% by weight for the peptide (hereinafter referred to as "PSF-6"). 
Xaa Xbb Xcc Xdd Xee Cys Pro Val Xff Xgg Lys Arg Leu Leu
1               5                   10
Xhh Val Val Val Val Val Val Leu Xii Val Val Val Ile Val
15                  20                  25
 
Gly Ala Leu Xjj Xkk Xll Xmm
30 35
 where Xaa does not exist or represents Phe or Leu, Xbb does not exist or represents Gly, Arg or Leu, Xcc does not exist or represents Ile, Xdd does not exist or represents Pro, Xee does not exist or represents Cys, Xff represents His or Asn, Xgg represents Leu or Ile, Xhh represents Val or Ile, Xii represents Ile, Leu or Val, Xjj does not exist or represents Leu, Xkk does not exist or represents Met, Xll does not exist or represents Gly, and Xmm does not exist or represents Leu.

Suitable examples of the choline phosphoglyceride in PSF-6 include 1,2-diacylglycero(3)-phosphocholines such as 1,2-dipalmitoylglycero-(3)-phosphocholine (also known as dipalmitoylphosphatidylcholine), 1,2-distearoylglycero-(3)-phosphocholine, 1-palmitoyl-2-stearoylglycero-(3)-phosphocholine and 1-stearoyl-2-palmitoylglycero-(3)-phosphocholine; 1-alkyl-2-acylglycero-(3)-phosphocholines such as 1-hexadecyl-2-palmitoylglycero-(3)-phosphocholine and 1-octadecyl-2-palmitoylglycero-(3)-phosphocholine; and 1,2-diacylglycero-(3)-phosphocholines such as 1,2-dihexadecyl-2-palmitoylglycero-(3)-phosphocholine. Although these compounds have optical isomers based on the asymmetric carbon atom at the 2-position of the glycerol residue, any of the D-, L- and DL-isomers may be used in this surfactant. In addition to the above-described pure choline phosphoglycerides, there can also be used mixtures of two or more 1,2-diacylglycero-(3)-phosphocholines having two acyl groups (preferably saturated acyl groups) of 12 to 24 carbon atoms, and mixtures of such a mixture and any of the above-described pure compounds.

Suitable examples of the acid phospholipid in PSF-6 include 1,2-diacyl-sn-glycero-(3)-phosphoric acid (also known as phosphatidic acid), 1,2-diacyl-sn-glycero-(3)-phospho-L-serine (also known as phosphatidylserine), 1,2-diacyl-sn-glycero-(3)-phospho-sn-glycerol (also known as phosphatidylglycerol) and 1,2-diacyl-sn-glycero-(3)-phospho-(1)-L-myo-inositol (also known as phosphatidylinositol). These compounds may be substituted by the same or difference acyl groups at the 1- and 2-positions. These acyl groups preferably have 12 to 24 carbon atoms.

Suitable examples of the fatty acid or its analogue in PSF-6 include free fatty acids, fatty acid alkali metal salts, fatty acid alkyl esters, fatty acid glycerol esters and fatty acid amides, and mixtures of two or more such compounds. Moreover, fatty alcohols and aliphatic amines are also suitable.

In PSF-6, calcium ions may be present at the time of suspension according to the need, in order to stabilize its therapeutic activity.

As used herein, the term "fatty acid or its analogue" is intended to comprehend fatty alcohols and aliphatic amines as described above.

Although suitable fatty acids are myristic acid, palmitic acid and stearic acid, palmitic acid is preferred. The preferred fatty acid alkali metal salt is sodium palmitate, the preferred fatty acid alkyl ester is ethyl palmitate, the preferred fatty acid glycerol ester is monopalmitin, and the preferred fatty acid amide is palmitic acid amide. The preferred fatty alcohol is hexadecyl alcohol, and the preferred aliphatic amine is hexadecylamine. The above-described choline phosphoglycerides, acid phospholipids and fatty acids or their analogues may be any of products isolated from animals or plants, semisynthetic products and chemically synthesized products, and commercially available products thereof can be used.

Although any of the above-described PSFs can used in the prophylactic and therapeutic agent of the present invention, it is desirable to use a PSF containing a protein, lipoprotein or peptide as a component.

The prophylactic and therapeutic agent of the present invention can be applied to viral diseases occurring in a region of the respiratory tract as a result of infection with viruses having envelope glycoproteins. Specific examples thereof include diseases caused by influenza virus, parainfluenza virus, respiratory syncytial virus (hereinafter referred to as "RS virus"), measles virus and mumps virus.

### BEST MODE FOR CARRYING OUT THE INVENTION

The prophylactic and therapeutic effects of PSFs on viral diseases will be described hereinbelow. The PSFs, viruses and Tryptase Clara used in the following tests are as follows:

### (PSFs)

The above-described PSF-1, PSF-2, PSF-3, PSF-4, PSF-5 and PSF-6 were chosen for use in the following tests. The particular PSFs used as typical examples had the respective compositions given below. In these compositions, all percentages are by weight. With regard to PSF-4 and PSF-5, commercially available products were used.

### PSF-1

| | |
|---|---|
| Phospholpid | 85.5% |
| Neutral fat | 2.4% |
| Total cholesterol | 0.1% |
| Free fatty acids | 8.2% |
| Carbohydrate | 0.2% |
| Protein | 0.7% |
| Water | 2.9% |

### PSF-2

| | |
|---|---|
| 1,2-Dipalmitoylglycero-(3)-phosphocholine | 69.8% |
| 1,2-Diacyl-sn-glycero-(3)-phospho-sn-glycerol | 17.5% |
| Palmitic acid | 8.7% |
| Lipoprotein extracted from cattle lung | 2.2% |
| Water | 1.8% |

### PSF - 3

| | |
|---|---|
| 1,2-Dipalmitoylglycero-(3)-phosphocholine | 67.9% |
| 1,2-Diacyl-sn-glycero-(3)-phospho-sn-glycerol | 22.6% |
| Palmitic acid | 9.1% |
| Water | 0.4% |

### PSF-6

| | |
|---|---|
| 1,2-Dipalmitoylglycero-(3)-phosphocholine | 67.2% |
| 1,2-Diacyl-sn-glycero-(3)-phospho-sn-glycerol | 22.4% |
| Palmitic acid | 9.0% |
| Water | 0.5% |
| Peptide represented by the following specific sequence | 0.9% |

Phe Gly Ile Pro Cys Cys Pro Val His Leu Lys Arg Leu Leu
1               5                   10
Ile Val Val Val Val Val Val Leu Ile Val Val Val Ile Val
15                  20                      25
Gly Ala Leu Leu Met Gly Leu
    30                  35

### (Viruses)

The viruses chosen for use in the following tests were Sendai virus (Z strain) and influenza virus (A/Aichi/2/68 H3N2). Sendai virus and influenza virus were used by growing each of them in the amnionic cavity of a developing hen's egg and suspending the resulting virus particles in a calcium-free phosphate buffer solution (hereafter referred to as "PBS(-)") at a concentration of 256 HAU/ml (i.e., hemagglutination units/ml).

### (Tryptase Clara)

Employing the method described in an article by Hiroshi Kido et al. (THE Journal of Biological Chemistry, Vol. 267, 1992), Tryptase Clara was prepared from rat lungs in the following manner.

Rat lungs were washed with physiological saline, minced and homogenized at pH 5.5, followed by centrifugation. The supernatant was obtained as a crude extract and purified by the following four chromatographic procedures. Specifically, the resulting crude extract was successively subjected to ion exchange column chromatography using a CM52 - cellulose column (trade name) and a CM52 - Sephadex column (trade name), whereby a fraction exhibiting activity against a color-producing substrate for the determination of trypsin activity, such as Boc-Gln-Ala-Arg-MCA, was collected. This collected fraction was subjected to chromatography using an arginine-Sepharose column (trade name) which is a specific adsorbent for serine proteases, whereby a trypsin-like enzyme was collected by specific adsorption. Finally, Tryptase Clara was isolated and purified by passing the resulting enzyme solution through a gel permeation column and collecting a fraction exhibiting activity against a color-producing substrate for the determination of trypsin activity. This Tryptase Clara was dissolved in PBS(-) at a concentration of 1 mg/ml and used in the tests.

### Tests for the Inhibition of Virus Infection and Multiplication

These tests were carried out by determining the cell infecting unit (CIU) of each virus, as described in the Journal of Virology, Vol. 8, 619-629, 1971, according to the following procedure.

20 µl of Tryptase Clara and 20 µl of each PSF were placed in a test tube and incubated at 0°C for 20 minutes. After 80 µl of Sendai virus or influenza virus was added, the mixture was incubated at 37°C for 20 minutes. Thereafter, the enzyme activity of Tryptase Clara was stopped by the addition of 10 µl of Aprotinin (trade name) as a protease inhibitor. Then, the mixture was cooled in ice water to obtain a virus reaction solution. For this purpose, PSF-1, PSF-2, PSF-3, PSF-4 and PSF-5 were used in the form of a suspension in PBS(-), and PSF-6 was used in the form of a suspension in a phosphate buffer solution containing a very small amount of calcium ions.

Next, rhesus monkey kidney cells were placed in an 8-chamber slide in an amount of 2 × 10⁵ cells per chamber and incubated for 2 to 3 days. Then, this slide was washed twice with PBS(-), inoculated with the virus reaction solution in an amount of 10 or 50 µl per chamber, and incubated in a CO₂ incubator at 35°C for 60 minutes. To the resulting culture solution, minimum essential medium containing 0.2% bovine serum albumin was added in an amount of 300 µl per chamber, followed by incubation at 37°C for 15 hours. After the medium was removed, the slide was washed once with PBS(-), fixed by immersion in acetone at 0°C for 20 minutes, and immunologically stained with fluorescein using antibodies. The antibodies used were anti-Sendai virus rabbit serum and anti-rabbit immunoglobulin goat immunoglobulin labeled with fluorescein isothiocyanate, or anti-influenza virus rabbit serum and anti-rabbit immunoglobulin goat immunoglobulin labeled with fluorescein isothiocyanate. The cell infecting unit was determined by counting fluorescent cells as infection-positive cells under a microscope of 400 magnifications and converting the cell count to the value corresponding to 1 ml of the virus reaction solution. The test results obtained with Sendai virus are shown in Table 1, and the test results obtained with influenza virus are shown in Table 2.

In both tables, control 1 shows the result obtained in the same manner as described above, except that the virus reaction solution was prepared by placing 80 µl of Sendai virus or influenza virus in a test tube without treating it with Tryptase Clara, incubating it at 37°C for 20 minutes, and cooling it with ice water. Control 2 shows the result obtained in the same manner as described above, except that the virus reaction solution was prepared by placing 20 µl of Tryptase Clara in a test tube, incubating it at 0°C for 20 minutes, adding thereto 80 µl of Sendai virus or influenza virus, incubating this mixture at 37°C for 20 minutes, stopping the enzyme activity of Tryptase Clara, and cooling the mixture with ice water. "PSF concentration" means the concentration of each PSF in PBS(-). In the case of PSF-6, however, the concentration is expressed as the value calculated by excluding the coexisting calcium ions.

**Table 1**

| Group | PSF concentration (mg/ml) | Cell infecting unit of Sendai virus, number of infection-positive cells per chamber (× 10⁴ CIU/ml) |
|---|---|---|
| Control 1 | - | 2 |
| Control 2 | - | 180 |
| Treated with PSF-1 | 0.232 | 25 |
| | 1.16 | 20 |
| | 5.80 | 13 |
| | 29.0 | 5.0 |
| Treated with PSF-2 | 0.232 | 6.4 |
| | 1.16 | 2.1 |
| | 5.80 | 1.5 |
| | 29.0 | 0.28 |
| Treated with PSF-3 | 0.244 | 3.6 |
| | 1.12 | 5.8 |
| | 5.60 | 4.9 |
| | 28.0 | 0.54 |
| Treated with PSF-4 | 29.0 | 8.4 |
| Treated with PSF-5 | 29.0 | 11 |
| Treated with PSF-6 | 0.096 | 18 |
| | 0.48 | 16 |
| | 2.40 | 4.7 |
| | 12.0 | 2.4 |

**Table 2**

| Group | PSF concentration (mg/ml) | Cell infecting unit of influenza virus, number of infection-positive cells per chamber (× 10⁴ CIU/ml) |
|---|---|---|
| Control 1 | - | 0.15 |
| Control 2 | - | 76 |
| Treated with PSF-1 | 0.232 | 24 |
| | 1.16 | 19 |
| | 5.80 | 9.1 |
| | 29.0 | 2.5 |
| Treated with PSF-2 | 0.232 | 17 |
| | 1.16 | 7.3 |
| | 5.80 | 1.9 |
| | 29.0 | 0.37 |
| Treated with PSF-3 | 0.244 | 27 |
| | 1.12 | 15 |
| | 5.60 | 8.0 |
| | 28.0 | 2.2 |
| Treated with PSF-4 | 29.0 | 7.9 |
| Treated with PSF-5 | 29.0 | 15 |
| Treated with PSF-6 | 0.096 | 18 |
| | 0.48 | 16 |
| | 2.40 | 1.4 |
| | 12.0 | 0.12 |

As is evident from Tables 1 and 2, all PSFs suppressed the increase of infected cells induced by Tryptase Clara, in a concentration-dependent manner. Thus, all PSFs were found to prevent infection and multiplication of the viruses.

### Tests for the Survival of Virus-Infected Rats

Using 3-weeks-old male CD(SD) rats (purchased from Charles River Japan Inc.) in groups of ten, these tests were carried out by administering each PSF once before virus infection (hereinafter referred to as "single treatment tests before infection") or repeatedly after virus infection (hereinafter referred to as "repeated treatment tests after infection"), and observing the rats to determine the number of days of survival of each rat. The virus chosen for these tests was Sendai virus (Z strain), and the PSFs chosen for these tests were PSF-1, PSF-2, PSF-3 and PSF-6. These PSFs were used in the form of a suspension in physiological saline.

Virus infection was effected by administering 2x104 plaque-forming units of Sendai virus pernasally to each rat under ether anesthesia. In the single treatment tests before infection, each PSF was endotracheally administered once 10 minutes before virus infection in a dose of 108 mg/kg for PSF-1, PSF-2 and PSF-3 or 44 mg/kg for PSF-6. In the repeated treatment tests after infection, each PSF was pernasally administered in a dose of 27 mg/kg for PSF-1, PSF-2 and PSF-3 or 12 mg/kg for PSF-6. This treatment was started immediately after infection and continued at intervals of 8 hours for a period of 4 days. In a control group, an equal volume of physiological saline was administered instead of a PSF. The results of the single treatment tests before infection are shown in Table 3, and the results of the repeated treatment tests after infection are shown in Table 4.

In both tables, "Number of days" means the number of days after virus infection, and "Number of surviving rats" means the number of rats surviving on a given day.

**Table 3**

| Group (10 rats per group) | Number of surviving rats in single treatment tests before infection | | | | | | | | | |
|---|---|---|---|---|---|---|---|---|---|---|
| | Number of days | | | | | | | | | |
| | 1 | 2 | 3 | 4 | 5 | 6 | 7 | 8 | 9 | 10 |
| Control | 10 | 10 | 10 | 10 | 8 | 4 | 1 | 0 | 0 | 0 |
| Treated with PSF-1 | 10 | 10 | 10 | 10 | 10 | 10 | 9 | 8 | 8 | 8 |
| Treated with PSF-2 | 10 | 10 | 10 | 10 | 10 | 10 | 9 | 7 | 6 | 6 |
| Treated with PSF-3 | 10 | 10 | 10 | 10 | 10 | 9 | 8 | 7 | 5 | 5 |
| Treated with PSF-6 | 10 | 10 | 10 | 10 | 10 | 10 | 9 | 8 | 7 | 7 |

**Table 4**

| Group (10 rats per group) | Number of surviving rats in repeated treatment tests after infection | | | | | | | | | |
|---|---|---|---|---|---|---|---|---|---|---|
| | Number of days | | | | | | | | | |
| | 1 | 2 | 3 | 4 | 5 | 6 | 7 | 8 | 9 | 10 |
| Control | 10 | 10 | 10 | 10 | 7 | 4 | 2 | 0 | 0 | 0 |
| Treated with PSF-1 | 10 | 10 | 10 | 10 | 10 | 10 | 10 | 10 | 10 | 10 |
| Treated with PSF-2 | 10 | 10 | 10 | 10 | 10 | 10 | 10 | 10 | 10 | 10 |
| Treated with PSF-3 | 10 | 10 | 10 | 10 | 10 | 10 | 10 | 10 | 10 | 10 |
| Treated with PSF-6 | 10 | 10 | 10 | 10 | 10 | 10 | 10 | 10 | 10 | 10 |

As is evident from Table 3, the single treatment tests before infection revealed that, in the control group, some animals began to die 5 days after infection and all animals died by day 8 (with a survival rate of 0%), whereas in the PSF-treated groups, 5-8 animals survived on day 10 (with a survival rate of 50-80%). Moreover, as is evident from Table 4, the repeated treatment tests after infection revealed that, in the control group, some animals began to die 5 days after infection and all animals died by day 8 (with a survival rate of 0%), whereas in the PSF-treated groups, all animals survived on day 10 (with a survival rate of 100%). This indicates that the PSFs very effectively prevented infection and multiplication of the virus. On the basis of these facts, all PSFs were found to be effective in preventing and inhibiting infection and multiplication of the virus.

### Postmortem Examination of the Lungs of Virus-Infected Rats

Using 3-weeks-old male CD(SD) rats in groups of seven, these tests were carried out by administering 2x104 plaque-forming units of Sendai virus pernasally to the rats, administering each PSF pernasally to the infected rats at intervals of 8 hours for a period of 4 days, sacrificing all rats 5 days after infection, excising the lungs, and observing the degree of injury of the lungs with the naked eye. The PSFs chosen for these tests were PSF-1, PSF-2, PSF-3 and PSF-6, which were used in the form of a suspension in physiological saline. Each PSF was administered in a dose of 27 mg/kg for PSF-1, PSF-2 and PSF-3 or 12 mg/kg for PSF-6. In a control group, an equal volume of physiological saline was administered instead of a PSF. The results thus obtained are shown in Table 5.

In Table 5, the degree of injury of the lungs is rated by expressing the proportion of a hepatized area (i.e., an area assuming a brown color) to the whole lung surface area by a score ranging from 0 to 4. Scores 0, 1, 2, 3 and 4 mean that the percentage of the hepatized area to the whole lung surface area was 0%, 1-25%, 26-50%, 51-75% and 76-100%, respectively.

**Table 5**

| Group (7 rats per group) | Score expressing the degree of injury of the lungs (number of rats) | | | | | Mean value |
|---|---|---|---|---|---|---|
| | 0 | 1 | 2 | 3 | 4 | |
| Control | | | | 1 | 6 | 3.86 |
| Treated with PSF-1 | 2 | 2 | 2 | 1 | | 1.29 |
| Treated with PSF-2 | 1 | 3 | 2 | 1 | | 1.43 |
| Treated with PSF-3 | 2 | 1 | 2 | 2 | | 1.57 |
| Treated with PSF-6 | 2 | 3 | 2 | 0 | | 1.00 |

It is evident from Table 5 that all PFSs hindered extension of the range of injury of the lungs due to virus infection. Thus, all PFSs were found to be effective in preventing infection and multiplication of the virus.

### Acute and Subacute Toxicity Tests

### (a) Acute toxicity

LD₅₀ values were determined by administering PSFs orally or intraperitoneally to 5-weeks-old male ICR mice and 5-weeks-old male Wistar rats. The PSFs chosen for these tests were PSF-1, PSF-2, PSF-3 and PSF-6. For mice, the LD₅₀ values were not less than 3 g/kg when administered orally and not less than 2 g/kg when administered intraperitoneally. For rats, the LD₅₀ values were not less than 4 g/kg when administered orally and not less than 2.5 g/kg when administered intraperitoneally.

### (b) Subacute toxicity

PSF-1, PSF-2 and PSF-3 were administered intraperitoneally to mature Wistar rats in a daily dose of 500 mg/kg for one month. After one month, no abnormalities were noted in the body weights of the rats and in visual and histological observations of the lungs and other principal organs.

### Dosage Regimen

The prophylactic and therapeutic agent for viral diseases in a region of the respiratory tract in accordance with the present invention contains a PSF in a dose of 0.001 to 500 mg, preferably 0.002 to 200 mg, for infants or 0.005 to 800 mg, preferably 0.01 to 400 mg, for adults. This dose is suspended in water or an electrolyte solution (e.g., physiological saline) or in such a liquid containing calcium ions according to the need, so as to give a concentration of 0.05 to 300 mg/ml, preferably 0.1 to 200 mg/ml. The resulting suspension is injected or sprayed into the respiratory tract before virus infection or after the onset of a viral disease.

The treatment is usually repeated 1 to 100 times, preferably 1 to 50 times, before virus infection, or 1 to 200 times, preferably 1 to 80 times, after the onset of a viral disease. The above-described dosage, method of administration and number of treatments may suitably be modified according to the condition or age of the patient, any therapy used in combination, and the degree of prevalence of the viral disease to be treated.

If desired, the prophylactic and therapeutic agent of the present invention may additionally contain pharmaceutical additives such as stabilizers, preservatives, tonicity agents, buffer agents and suspending agents, and/or drugs such as bronchodilators, antitussives, antibiotics, synthetic antibacterial agents and antiviral agents. The prophylactic and therapeutic agent of the present invention is preferably in the form of a powder to be suspended before use, or an aerosol. It is filled into vials, ampules or other hermetic containers to form sterile products.

### INDUSTRIAL APPLICABILITY

Generally, viral diseases in a region of the respiratory tract tend to occur epidemically and we are often informed of their prevalence. Accordingly, the prophylactic and therapeutic agent for viral diseases in a region of the respiratory tract in accordance with the present invention can be used as a prophylactic agent for an epidemic disease after information about its prevalence is given, and as a therapeutic agent for an epidemic disease after its onset. In particular, the prophylactic and therapeutic agent of the present invention can be effectively applied to viral diseases caused by viruses which have envelope glycoproteins and are capable of infection and multiplication in a region of the respiratory tract, such as influenza virus, parainfluenza virus, RS virus, measles virus and mumps virus.

LIST OF SEQUENCES

SEQ ID NO: 1

SEQUENCE LENGTH: 35

SEQUENCE TYPE: Amino acid

TOPOLOGY: Linear

MOLECULE TYPE: Peptide

SEQUENCE DESCRIPTION:

Phe Gly Ile Pro Cys Cys Pro Val His Leu Lys Arg Leu Leu Ile

1               5                   10                  15

Val Val Val Val Val Val Leu Ile Val Val Val Ile Val Gly Ala

                20                  25                  30

Leu Leu Met Gly Leu

                35

## Claims

1. A prophylactic and therapeutic agent for viral diseases in a region of the respiratory tract which contains a pulmonary surfactant.

2. A prophylactic and therapeutic agent as claimed in claim 1 wherein the pulmonary surfactant contains a phospholipid consisting essentially of a phosphatidyl-choline or a choline phosphoglyceride, in an amount of not less than 40% by weight based on the total dry weight of the pulmonary surfactant.

3. A prophylactic and therapeutic agent as claimed in claim 2 wherein the pulmonary surfactant contains a specific protein, lipoprotein or peptide.

4. A prophylactic and therapeutic agent as claimed in claim 3 wherein the pulmonary surfactant is a surface-active substance comprising phospholipid, neutral fat, total cholesterol, free fatty acids, carbohydrate and protein that are present in the lung tissue of a mammal, the weight percentages of these components based on the total dry weight of the substance being 68.6-90.7% for the phospholipid, 0.3-13.0% for the neutral fat, 0.0-8.0% for the total cholesterol, 1.0-27.7% for the free fatty acids, 0.1-2.0% for the carbohydrate, and 0.0-3.5% for the protein.

5. A prophylactic and therapeutic agent as claimed in claim 4 wherein the surface-active substance comprises 85.5% by weight of phospholipid, 2.4% by weight of neutral fat, 0.1% by weight of total cholesterol, 8.2% by weight of free fatty acids, 0.2% by weight of carbohydrate, and 0.7% by weight of protein.

6. A prophylactic and therapeutic agent as claimed in claim 3 wherein the pulmonary surfactant consists essentially of a choline phosphoglyceride, an acid phospholipid, a fatty acid or its analogue, and a lipoprotein derived from the lung of a mammal, the contents of these components based on the total weight of the pulmonary surfactant being 50.6-85.0% by weight for the choline phosphoglyceride, 4.5-37.6% by weight for the acid phospholipid, 4.6-24.6% by weight for the fatty acid or its analogue, and 0.1-10.0% by weight for the lipoprotein.

7. A prophylactic and therapeutic agent as claimed in claim 6 wherein the surfactant comprises 69.8% by weight of 1,2-dipalmitoylglycero-(3)-phosphocholine, 17.5% by weight of 1,2-diacyl-sn-glycero-(3)-phospho-sn-glycerol, 8.7% by weight of palmitic acid, and 2.2% by weight of a lipoprotein extracted from cattle lung.

8. A prophylactic and therapeutic agent as claimed in claim 2 wherein the pulmonary surfactant consists essentially of a choline phosphoglyceride, an acid phospholipid and a fatty acid or its analogue, the contents of these components based on the total weight of the pulmonary surfactant being 53.9-87.8% by weight for the choline phosphoglyceride, 4.8-38.2% by weight for the acid phospholipid, and 7.0-26.2% by weight for the fatty acid or its analogue.

9. A prophylactic and therapeutic agent as claimed in claim 8 wherein the surfactant comprises 69.7% by weight of 1,2-dipalmitoylglycero-(3)-phosphocholine, 22.6% by weight of 1,2-diacyl-sn-glycero-(3)-phospho-sn-glycerol, and 9.1% by weight of palmitic acid.

10. A prophylactic and therapeutic agent as claimed in claim 3 wherein the pulmonary surfactant is Alveofact.

11. A prophylactic and therapeutic agent as claimed in claim 2 wherein the pulmonary surfactant is Exosurf.

12. A prophylactic and therapeutic agent as claimed in claim 3 wherein the pulmonary surfactant is a pulmonary surface-active substance consisting essentially of a choline phosphoglyceride, an acid phospholipid, a fatty acid or its analogue, and a peptide represented by the following specific sequence, the contents of these components based on the total dry weight of the pulmonary surfactant being 50.6-85.0% by weight for the choline phosphoglyceride, 4.5-37.6% by weight for the acid phospholipid, 4.6-24.6% by weight for the fatty acid or its analogue, and 0.1-5.0% by weight for the peptide. 
Xaa Xbb Xcc Xdd Xee Cys Pro Val Xff Xgg Lys Arg Leu Leu
1               5                   10
Xhh Val Val Val Val Val Val Leu Xii Val Val Val Ile Val
15                  20                  25
Gly Ala Leu Xjj Xkk Xll Xmm
    30                  35
 where Xaa does not exist or represents Phe or Leu, Xbb does not exist or represents Gly, Arg or Leu, Xcc does not exist or represents Ile, Xdd does not exist or represents Pro, Xee does not exist or represents Cys, Xff represents His or Asn, Xgg represents Leu or Ile, Xhh represents Val or Ile, Xii represents Ile, Leu or Val, Xjj does not exist or represents Leu, Xkk does not exist or represents Met, Xll does not exist or represents Gly, and Xmm does not exist or represents Leu.

13. A prophylactic and therapeutic agent as claimed in claim 12 wherein the pulmonary surface-active substance comprises 67.2% by weight of 1,2-dipalmitoylglycero-(3)-phosphocholine, 22.4% by weight of 1,2-diacyl-sn-glycero-(3)-phospho-sn-glycerol, 9.0% by weight of palmitic acid, and 0.9% by weight of a peptide represented by the following specific sequence:

Phe Gly Ile Pro Cys Cys Pro Val His Leu Lys Arg Leu Leu
1               5                   10
Ile Val Val Val Val Val Val Leu Ile Val Val Val Ile Val
15                  20                  25
Gly Ala Leu Leu Met Gly Leu
    30                  35

14. A prophylactic and therapeutic agent as claimed in any one of claims 1 to 13 wherein the causative virus is a virus which has envelope glycoproteins and is capable of infection and multiplication in a region of the respiratory tract.

15. A prophylactic and therapeutic agent as claimed in claim 14 wherein the region of the respiratory tract is a bronchial region.

16. A prophylactic and therapeutic agent as claimed in claim 15 wherein the virus is influenza virus, parainfluenza virus, respiratory syncytial virus, measles virus or mumps virus.
